# EUROPEAN PATENT APPLICATION

(11) **EP 3 108 810 A1**
(43) Date of publication of application: **28.12.2016**
(21) Application number: 15173353.2
(22) Date of filing: 23.06.2015
(51) Int. Cl.: A61B 5/145, A61B 5/07

(54) **INGESTIBLE DEVICE FOR MEASURING GLUCOSE CONCENTRATION**

(71) Applicant: Valtronic Technologies (Holding) SA, 1343 Les Charbonnières (CH)
(72) Inventor: Lepple-Wienhues, Albrecht, 25300 Pontarlier (FR)
(74) Representative: Patentanwälte Ruff, Wilhelm, Beier, Dauster & Partner mbB

(57) **Abstract**

The invention refers to an ingestible device for measuring glucose concentration in the small intestine, and also to a method for measuring the glucose concentration in the small intestine, using this inventive device.

The ingestible device comprises the following components, namely at least one retainer for temporarily fixing the device in the small intestine, at least one glucose sensor connected to the retainer, at least one power source, and at least one wireless data transmitter/date receiver.

The method according to the invention comprises the following steps, namely,
- activating the device,
- fixing the device in the small intestine via a retainer,
- measuring data related to glucose concentration inside the small intestine via a glucose sensor, and
- transmitting data related to the glucose concentration to a receiver located outside the small intestine, in particular for determining or predicting the glucose level in the blood stream of the subject.

## Description

### FIELD OF THE INVENTION

This invention relates to an ingestible device for measuring glucose concentration. Further, the invention relates to a method for measuring the glucose concentration, wherein the inventive device is ingested by a human subject or animal subject.

Finally, the invention relates to a sensor for measuring the glucose concentration in the small intestine.

### BACKGROUND TO THE INVENTION

Diabetes is a very frequent disease affecting many millions of patients worldwide. Underlying the disease is an absolute (type 1) or relative (type 2) lack of insulin. This hormone is required to keep the blood glucose level in the small concentration range that is required for energizing the cells in the body while keeping blood glucose below dangerous, toxic levels. In diabetic patients, it is often required several times per day to have the patient inject insulin, estimate glucose uptake with each meal and measure glucose blood concentration frequently by drawing a drop of blood and using a blood glucose meter. All these measures need to be tightly controlled and adjusted by the patient and caretakers several times a day, because both over-dosing as well as under-dosing insulin may lead to severe health complications.

In the past three decades, there were many attempts to replace the discrete finger pricking based blood glucose measurements by continuously measuring devices. For measurements in the blood stream no satisfactory working solution has been developed up to now.

In the last few years, several devices have been approved that measure the glucose levels in the dermal interstitial fluid. However, the disadvantage of this approach lies in the delay of interstitial glucose levels that may be typically in the range of 10 to 15 minutes, creating a "blind" time interval during which glucose levels can reach alarming values e.g. after a meal or after injecting insulin. This can lead e.g. to hyperglycemic shock before the measured values drop below a critical threshold with potential fatal results.

Furthermore, attempts to measure glucose in secreted bodily fluids like tears, urine, saliva etc. have been unsuccessful due to active glucose reabsorption and a subsequently very low glucose concentration in secreted fluids and the lack of relevant correlation with blood glucose levels.

All attempts to monitor glucose levels continuously in body fluids are hindered by fouling of the sensors, leading to the requirement for frequent sensor replacement and recalibration. Therefore, a disposable sensor that only needs to be accurate for several hours is highly desirable.

In addition, it has proven difficult for patients and caretakers to precisely measure or estimate the carbohydrate content of meals and their efficient digestion into glucose in a given individual patient and meal. Generally, carbohydrate content of meals is roughly estimated with a great margin of error.

Under normal circumstances every single glucose molecule entering the blood stream is being absorbed and derived from digested carbohydrates in the small intestine. The bulk of glucose absorption after a meal is taking place in the duodenum. Furthermore, the resorption of glucose in the small intestine is almost complete.

### OBJECT OF THE INVENTION

The object of the present invention is to overcome said limitations of the prior art. The invention should create the possibility to determine the glucose uptake by a patient with each food intake as early and continuously as possible, in order to have a reliable overview about the glucose level in the blood. Then, it will be possible to administer insulin doses exactly as needed by the patient.

### SUMMARY OF THE INVENTION

For solving this object and other objects, the present invention provides an ingestible device for measuring glucose concentration in the small intestine, as defined in independent claim 1. Preferred embodiments of this device are defined in the claims dependent from claim 1. A sensor for measuring the glucose concentration in the small intestine, being a part of the inventive device or being incorporated into the inventive device is defined in independent claim 17.

Further, the present invention provides a method for measuring the glucose concentration in the small intestine, wherein the inventive device is ingested by a human subject or animal subject. The steps of this inventive method are defined in independent claim 12. Preferred embodiments of this inventive method are shown in the method claims dependent from claim 12.

All features of all claims are hereby incorporated into this description by explicit reference to the claims.

### DETAILED DESCRIPTION OF THE INVENTION

According to the invention, the ingestible device comprises at least the following components, namely,
- at least one retainer for temporarily fixing the device in the small intestine,
- at least one glucose sensor connected to the retainer,
- at least one power source, and
- at least one wireless data transmitter/data receiver.

Preferably, the glucose sensor is connected to the retainer via at least one elongated connecting element.

According to this invention, the term "retainer" means any device or equipment which is capable to fix the inventive device for a certain time in the small intestine. As a consequence, the retainer is something like an anchoring element temporarily fixing the device or at least part of the components of the device, in particular the glucose sensor, in the small intestine or relative to the small intestine.

The term "glucose sensor" means any device which is capable of measuring any value related to the glucose concentration within the human or animal body, namely in the small intestine. The term "power source" means any component supplying energy to the other components of the inventive device for fulfilling their corresponding function. Preferably, the power source is a galvanic cell, in particular a battery.

A "data transmitter/data receiver" is an electronic component of the inventive device for receiving data from outside the device and/or for transmitting data from the device. Normally, such a component of the inventive device will include an antenna.

The "small intestine" is the part of the gastrointestinal tract (GI tract) between the stomach and the large intestine. The small intestine is composed by the duodenum, jejunum and ileum. The major part of the digestion and absorption of food takes place in the small intestine.

In a preferred embodiment, the inventive device further comprises a casing which incorporates (houses) at least part of the components of the device. In particular, all of the components of the device are housed in this casing. The casing can have the form of a capsule or pill for being swallowed by the subject/patient.

In this context, it is preferred that all components of the inventive device are combined in a (preferably small) casing before this device is ingested by the corresponding subject, e.g. a patient. Therefore, it is easy for the patient to swallow the device. However, in its operating modus, the inventive device releases at least part of the components in order to measure the glucose concentration in the small intestine. This aspect of the present invention will be explained in more detail later.

Further, it is preferred according to the present invention that the device has a modular construction in which the components are at least partly separable from each other. This is helpful when the device after operation is to be excreted from the small intestine and the body of the corresponding subject/patient.

In other preferred embodiments, the inventive device is characterized by the feature that at least parts of the components of the device are made from a digestible material. Such a measure is also helpful in removing the device from the small intestine and the body of the corresponding subject/patient.

As mentioned above, the retainer component of the inventive device has kind of an anchoring function in order to temporarily fix the device in the small intestine. As a consequence, different designs of the retainer are possible according to the invention. However, it is preferred that the retainer is expandable or inflatable. This means that the retainer in its operation mode is expanded or inflated compared to its non-operation mode. In this context, the retainer preferably is housed in a casing before the inventive device is swallowed by the corresponding subject, and after activating the retainer to its operation mode the retainer is expanded or inflated for temporarily fixing the device in the small intestine or relative to the small intestine.

In this context, it is further preferred if the retainer in the inventive device also is reversibly collapsible or deflatable. This is helpful for removing the device from the small intestine and the body of the subject/patient.

Referring especially to the last paragraphs, it is preferred that the retainer comprises a balloon (as a structural element) or that the retainer comprises a parachute or an umbrella (as a structural element).

As mentioned above, the glucose sensor being a component of the ingestible device can be any sensor which is capable of measuring a value which is related to glucose concentration. As a consequence, it is possible that the glucose sensor is an optical sensor, an electronic sensor, a chemical sensor or an enzymatic sensor or a combination of said sensors. From all these sensors, it is preferred to use an optical sensor as a component in the inventive device.

As also mentioned above, the glucose sensor of the inventive device is connected to the retainer of the inventive device preferably via at least one elongated connecting element. In this context, it is preferred that this connecting element comprises at least one tether. This allows a flexible connection between the glucose sensor and the retainer.

In this context, it is also possible to connect the sensor to the retainer in a detachable manner. Then, it is possible to separate the sensor from the retainer, if necessary, e.g. for removing the corresponding parts from the small intestine and the body of the corresponding subject/patient.

Further, it is preferred that the inventive device also comprises an actuating element which activates (directly or indirectly) at least a part of the functions of the device. Such an actuating element can be responsible for activating the fixing function of the retainer and/or the measuring function of the glucose sensor. In this context, it is preferred that the actuating element is provided for activating the power source. Then, following activation of the power source other functions of the inventive device can be activated sequentially e.g. in preset time intervals.

The actuating element itself can be activated by a signal from outside the body of the subject/patient, e.g. by a radio or acoustic signal. Further, the actuating element can be activated after a certain predefined time during the ingestion phase of food in the body. Alternatively, the actuating element can be activated by the acidic pH-value in the stomach, by temperature, by humidity or by a combination of these parameters.

In a further preferred embodiment of the device the sensor is mounted or connected to a component that ensures close contact and/or vicinity of said sensor to the mucosa, wherein preferably said component has the function of an anchoring element fixing the component to the mucosa at least temporarily.

As mentioned above, the invention also comprises a method for (preferably continuously) measuring the glucose concentration in the small intestine, wherein the inventive device as already described is ingested by a human subject or animal subject. The inventive method comprises the following steps of
- activating the device,
- fixing the device in the small intestine via a retainer,
- measuring data related to glucose concentration inside the small intestine via a glucose sensor, and
- transmitting data related to the glucose concentration to a receiver located outside the small intestine, in particular located outside the body of the subject/patient.

The activation of the device is preferably performed via activating a power source. After activating the power source, it is preferred that the other functions of the inventive device (fixation in the small intestine, measurement of data related to glucose concentration and transmittance of these data) are initiated and run automatically.

Fixation of the device in the small intestine via the retainer particularly takes place at the pylorus between stomach and duodenum.

The measurement of data related to glucose concentration preferably takes place inside the duodenum and/or the jejunum and/or the ileum but further preferred inside the duodenum.

The transmitted data related to the glucose concentration are preferably used for determining or predicting the glucose level in the blood stream of the subject/patient.

Normally, according to the inventive method, the device is fixed in the small intestine for hours, in particular for 1 to 4 h, preferably for 1 to 2 h. During this fixation time of the device in the small intestine, it is possible to continuously measure data related to glucose concentration inside the small intestine via a glucose sensor, and, therefore, to determine or predict the glucose level in the blood stream of the corresponding subject/patient.

In preferred embodiments, the inventive method further comprises the step of removing the inventive device from the small intestine and from the body of the subject/patient by (preferably spontaneous) passage through the gastrointestinal tract of the subject/patient and excretion. This removing step can be initiated automatically, e.g. after some hours, by releasing the retainer from its fixation point, e.g. from the pylorus. Such release can be performed e.g. by collapsing or deflating a balloon-like or parachute-like or umbrella-like retainer. Furthermore, the device or parts of the device can be made from digestible materials, allowing for disintegration of the device or parts of the device for facilitated excretion.

According to preferred embodiments of the inventive method, the glucose concentration is measured within the lumen of the small intestine, preferably within the lumen of the duodenum. As a consequence, the glucose concentration is measured within the chyme present in the lumen of the small intestine.

It is further preferred according to the inventive method that the glucose concentration is measured within the mucosa of the small intestine, in particular within the mucosa of the duodenum, or within the physiological layer adjacent to the mucosa of the small intestine, preferably adjacent to the mucosa of the duodenum. With these embodiments, the glucose concentration is not measured within the bulk of the chyme, but more closely to the tissue or in the tissue of the body of the subject/patient. For these embodiments the use of inventive devices is preferred which comprise sensors mounted or connected to a component ensuring close contact/vicinity of said sensor to the mucosa (mentioned above).

It is further preferred according to the inventive method that in addition to the glucose concentration or even as an alternative to the glucose concentration, the concentration of other sugar molecules is measured, preferably the concentration of other monosaccharides like fructose and galactose or of disaccharides like sucrose and lactose. In another preferred embodiment the concentration of polysaccharides like starch is being measured, in addition or as an alternative.

As already mentioned, it is preferred with the inventive method that the data related to the glucose concentration measured inside the small intestine, preferably measured inside the duodenum, are used for determining or predicting the glucose level in the blood stream of the subject/patient. As a consequence, those data in the inventive method preferably can be used for controlling an insulin therapy of the subject/patient. In a preferred embodiment those data can be used for a closed-loop control of insulin administration, e.g. connecting to an insulin pump.

The inventive method with all embodiments described above can also be understood as use of the inventive device with all its embodiments for (preferably continuously) measuring glucose concentration in the small intestine.

Finally, the invention comprises a sensor for measuring the glucose concentration in the small intestine. This sensor is characterized that it is a part of the ingestible device as described or that it is incorporated into an ingestible device as described.

To summarize, the present invention is solving the problems of the prior art by measuring glucose levels in the lumen and/or mucosa of the upper intestine, i.e. the location where glucose is being released and absorbed into the blood stream after a meal. The gastric pylorus passes chyme containing carbohydrates into the duodenum in portions. Inside the duodenum, several enzymes cleave carbohydrates down to the monosaccharide level. The monosaccharides, most notably glucose, are then actively transported across the muscosal barrier into the blood stream. Therefore, the luminal glucose concentration in the duodenum and upper ileum will initially rise due to digestion and then decline due to absorption. When the glucose level becomes low, the pylorus will release another bolus of chyme for repeating the process, until after 1-3 hours the entire meal has been processed.

By measuring the profile of glucose concentrations in the lumen and/or mucosa during this process it is possible to calculate the elimination rate from the chyme which is equivalent to the blood uptake rate of glucose. It is also possible to calculate the amount of carbohydrates by observing the initial rise in glucose concentration. In contrast to existing technology, this allows to predict blood glucose levels rather than react to time-delayed measurements like finger-prick blood glucose testing or subcutaneous interstitial fluid readings.

This early warning intestinal glucose monitoring has the potential to greatly reduce or even eliminate the need for blood testing or interstitial recordings and to control the blood glucose levels in a subject/patient more accurately and efficiently than existing methods. Finally, the glucose monitoring data of the device can even control insulin administering devices like insulin pumps in order to create a feed-back loop mimicking the function of an intact insulin secreting apparatus in the pancreas.

Further advantages and features of the invention will become clear from the following description of the drawings in conjunction with the dependent claims. The individual features can be realized either singularly or separately in combination in one embodiment of the invention. The drawings merely serve for illustration and better understanding of the invention and are not to be understood as in any way limiting the invention.

The figures schematically show:
- Fig. 1: an illustration of the inventive device and of its function according to the inventive method, and
- Fig. 2: a typical pattern of glucose concentration in duodenal chyme to be measured with the inventive device.

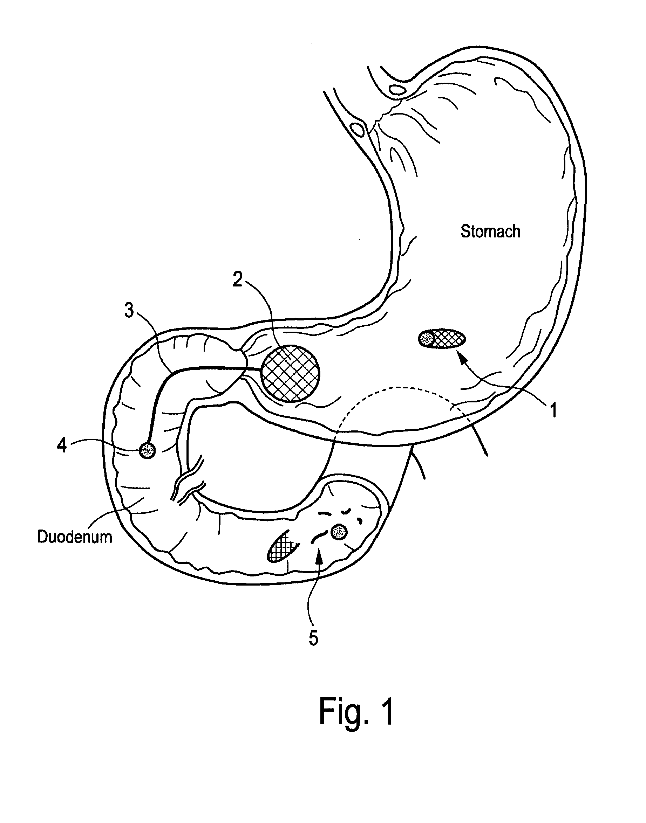

Fig. 1 illustrates the upper part of the gastrointestinal tract schematically, namely the stomach and the duodenum. The opening between stomach and duodenum is the pylorus (pyloric sphincter).

Within the stomach, Fig. 1 shows the inventive device 1 in its non-operating modus which is characterized by a casing housing all the necessary components of the inventive device. This casing is designed in the form of a capsule or pill which can be easily swallowed by the respective subject/patient.

All components and features described below in the context of Fig. 1 cannot only be realized with this specific embodiment but also with all other embodiments of the present invention.

According to the embodiment of the inventive device 1 shown in Fig. 1, this device comprises:
- a casing,
- a power source/power supply,
- a (data) transmitter connected with the power source,
- an antenna connected with the transmitter,
- an activator configured to activate the power source,
- possibly an electrical coupling element connected with the transmitter,
- a sensor for sensing glucose,
- a retainer/anchor component to retain/fix the device at least temporarily at the pylorus or the pyloric region, and
- electrical and/or mechanical connecting elements between the retainer and the sensor.

The sensor may be a glucose sensor out of a group consisting of an optical enzyme based sensor, an optical near infrared absorption sensor, an amperometric enzymatic sensor or a combination thereof. The power supply may comprise a battery. The transmitter may be configured to transmit data to a remote receiver electromagnetically or acoustically.

The capsule body may further comprise a sensor and/or trigger for temperature and/or pH and/or humidity. The device may further comprise a processor connected with the sensor. The sensor may be an analog sensor that provides an output voltage in response to stimuli. The modular sensing component may further comprise an analog-digital converter configured to convert an analog signal from the sensor to a digital signal. The retainer/anchor component and the sensor component may be mechanically and electrically attached to the capsule body prior to ingestion of the capsule by a subject and may be released upon ingestion by pH, temperature, humidity, or a combination thereof. The transmitter may send the measurements to a receiver located outside of the gastrointestinal tract of the subject by electromagnetic or acoustic waves.

Further, Fig. 1 also shows the situation in which the inventive device 1 is temporarily fixed at the pylorus/pyloric region via retainer 2 which is expanded after the casing of the inventive device 1 is opened and/ or the components are being separated after activation of the inventive device 1. Retainer 2 is connected to glucose sensor 4 via connecting element 3, e.g. via at least one tether. Therefore, it is possible to measure the glucose concentration in the duodenum with glucose sensor 4.

Finally, Fig. 1 shows the situation in which the components of the inventive device 1 are spontaneously removed from the small intestine and the body of the subject by peristaltic passage through the gastrointestinal tract of the subject. Parts of the device and / or its components are disintegrated, e.g. by long term exposure to digestive enzymes. Due to the modular construction of the inventive device 1, the components of the device are separated from each other and the remaining parts 5 of device 1 are removed from the small intestine and afterwards from the body of the subject.

A brief overview of the device function is provided as follows:
A patient swallows capsule (inventive device 1) together with a meal where all processing components are closely and compactly packed in miniaturized form to facilitate ingestion, e.g. by an acid-solvable casing. Upon reaching the warm, acidic and wet environment of the stomach, the electrical power is activated, the anchor element retainer 2 released and expanded, and the retainer 2 subsequently lodges itself in the pylorus sphincter where it cannot pass due its expanded physical size. Simultaneously or subsequently, the tethered connecting elements 3 that are smaller will be propelled into the duodenum by peristaltic expulsion of chyme from the stomach, their movement being restricted by the length of the tether 3 to the retainer 2. In this position, the sensor element 4 will continuously measure luminal and/or mucosal glucose concentration and the transmitter (not shown in Fig. 1) will send the data to a receiver outside the gastrointestinal tract.

These measurement data will allow a close estimate and/ or prediction of the changes of blood glucose level resulting from absorption and the insulin dosage scheme required to keep those levels within a physiological range. The algorithms for these calculations may be calibrated for an individual patient using blood glucose recordings around meals and insulin administration.

After 1 to 3 hours, when the entire meal has been digested, the expandable retainer 2 will collapse, release and pass the pyloric sphincter. The other tethered elements 3 will also release, and the elements 5 of the device will be transported towards the rectum and eventually be excreted.

The procedure will be repeated with each and every meal thus allowing for a complete quantitative and time-resolved monitoring of glucose uptake by the patient.

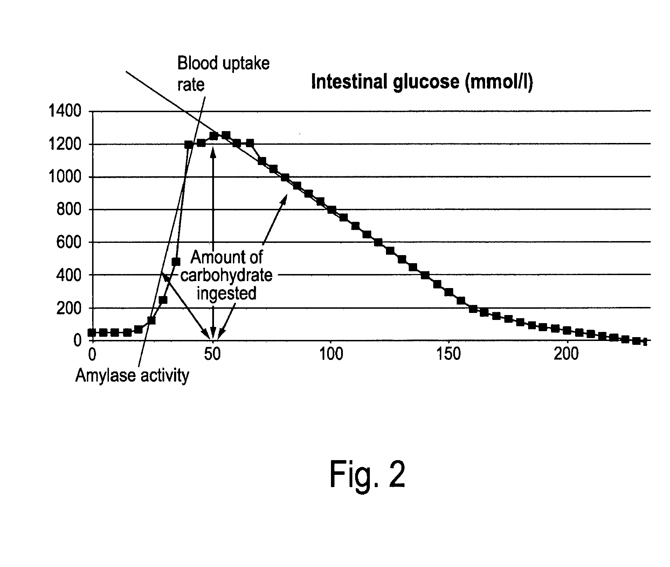

Fig. 2 shows a typical pattern of glucose concentration in the duodenal chyme as explained earlier. After opening of the pylorus, a bolus of chyme enters the duodenum. Due to the activity of enzymes and the corresponding enzymatic cleavage of carbohydrates, glucose levels are increasing initially. This increase is followed by a decline of glucose levels due to absorption of glucose into the blood stream.

With the inventive device and the inventive method, it will be possible to monitor the course of the glucose level continuously, thereby determining or predicting the glucose level in the blood stream of the corresponding subject. This will result in a corresponding control of an insulin therapy of this subject.

## Claims

1. Ingestible device (1) for measuring glucose concentration in the small intestine, comprising at least the following components, namely
- at least one retainer (2) for temporarily fixing the device in the small intestine,
- at least one glucose sensor (4) connected to the retainer (2), preferably via at least one elongated connecting element (3),
- at least one power source, and
- at least one wireless data transmitter/data receiver.

2. Device according to claim 1, further comprising a casing enclosing at least part of the components of the device, in particular all of the components of the device.

3. Device according to claim 1 or claim 2, **characterized in that** the device has a modular construction in which the components can be at least partially separated from each other.

4. Device according to one of the preceding claims, **characterized in that** at least parts of the components of the device are made from digestible materials.

5. Device according to one of the preceding claims, **characterized in that** the retainer is expandable or inflatable, and wherein the retainer preferably is also reversibly collapsible or reversibly deflatable.

6. Device according to one of the preceding claims, **characterized in that** the retainer is or comprises a balloon.

7. Device according to one of claims 1 to 5, **characterized in that** the retainer is or comprises or resembles a parachute or an umbrella.

8. Device according to one of the preceding claims, **characterized in that** the glucose sensor is an optical sensor, an electronic sensor, a chemical sensor or an enzymatic sensor, preferably an optical sensor, or a combination of said sensors.

9. Device according to one of the preceding claims, **characterized in that** the elongated connecting element is or comprises at least one tether.

10. Device according to one of the preceding claims, further comprising an actuating element which activates functions of the device, in particular the fixing function of the retainer and/or the measuring function of the glucose sensor.

11. Device according to claim 10, **characterized in that** the actuating element is provided for activating the power source.

12. Method for measuring the glucose concentration in the small intestine, wherein the device according to one of the preceding claims is ingested by a human subject or animal subject, with the following steps:
- activating the device (1), in particular via activating a power source,
- fixing the device in the small intestine via a retainer (2), in particular at the pylorus between stomach and duodenum,
- measuring data related to glucose concentration inside the small intestine via a glucose sensor (4), in particular inside the duodenum and/or the jejunum and/or the ileum, and
- transmitting data related to the glucose concentration to a receiver located outside the small intestine, in particular located outside the body of the subject, in particular for determining or predicting the glucose level in the bloodstream of the subject.

13. Method according to claim 12, further comprising the step of removing the device from the small intestine and the body of the subject by, preferably spontaneous, passage through the gastrointestinal tract of the subject.

14. Method according to claim 12 or claim 13, **characterized in that** the glucose concentration is measured within the lumen of the small intestine, preferably within the lumen of the duodenum.

15. Method according to one of claims 12 to 14, **characterized in that** the glucose concentration is measured within the mucosa of the small intestine, in particular within the mucosa of the duodenum, or within the physiological layer adjacent to the mucosa of the small intestine, preferably adjacent to the mucosa of the duodenum.

16. Method according to one of claims 12 to 15 **characterized in that** the data related to the glucose concentration measured inside the small intestine, preferably measured inside the duodenum, are used for controlling an insulin therapy of the subject.

17. Sensor for measuring the glucose concentration in the small intestine, **characterized in that** it is a part of the ingestible device according to one of claims 1 to 11 or that it is incorporated into an ingestible device according to one of claims 1 to 11.
